(19)

Europäisches
Patentamt
European
Patent Office
Office européen
des brevets

(11) **EP 3 876 236 A1**

(12) **EUROPEAN PATENT APPLICATION**

(43) Date of publication:
08.09.2021  Bulletin 2021/36

(51) Int Cl.:
*G16C 20/70* *(2019.01)*    *G16C 20/80* *(2019.01)*

(21) Application number: 20161025.0

(22) Date of filing: 04.03.2020

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Merck Patent GmbH
64293 Darmstadt (DE)**

(72) Inventor: **The designation of the inventor has not
yet been filed**

(54) **EXTRACTING CHEMICAL STRUCTURES FROM DIGITIZED IMAGES**

(57)    The present disclosure relates to methods and devices for extracting at least one chemical structure from a template by assembling from the part-based representations an identical representation or an equivalent representation of the chemical structure.

FIG. 1

EP 3 876 236 A1

## Description

The present disclosure relates to methods and apparatuses for extracting a chemical structure.

In the scientific literature, there is a tremendous amount of information about the interaction of small molecules with specific targets, the influence of small molecules on biochemical pathways, the phenotypic effects of small molecules in different cell types, as well as the relationship of small molecules, targets, pathways and phenotypes to disease processes. However, much of this information has yet to be compiled in the form that would allow using a molecule's chemical structure as an input to search for its potential relevance in a specific physiological, pathological or therapeutic area of interest.

Two examples of information resources linking chemical structures with biomedical targets, pathways and phenotypes are PubMed - the database of the scientific literature corpus - and PubChem- a publicly available database of over 19 million chemical structures, each of which can have a cross-reference link to similar structures, bio-assay data, and bio-activity descriptions. If these resources can be used to construct a universal database encompassing all known chemical structures with links to specific targets, biochemical pathways, disease states and potential therapeutic applications, a powerful new tool for both biomedical research and drug discovery would emerge.

In general, one can envision two ways to parse scientific articles for chemical information: by searching for names or structure diagrams of chemical agents. The chemical structure diagrams in scientific articles are typically drawn manually using a program such as Chem-Draw, ISIS/Draw, DrawIt, MarvinSketch and ACD/Chem-Sketch. Once a structure is drawn, the structural description can be translated into a computer readable format, such as ISIS/Draw, ChemDraw Exchange CDX, MOLfile, IUPAC International Chemical Identifier InChI, SMILES or ROSDAL formats, which describes the atoms, bond orders, and connectivity patterns of atoms in molecules. However, the diagrams of chemical molecules in scientific journals and reference books are encoded as digitized images (e.g. BMP, JPG, TIFF, PNG or GIF), which in turn are embedded within lines of text in a form that is not readily translatable into a computer readable format. Therefore, most references to chemical agents in scientific research articles cannot be easily linked to other repositories of scientific knowledge, and, are thus not amenable for analysis or searching using cheminformatic software.

Further, most scientific and patent documents dealing with chemistry are describing molecular structures either with systematic names or with graphical images of Lewis structures. The latter method poses inherent problems in the automated processing that is needed when the number of documents ranges in the hundreds of thousands or even millions since such human-readable graphical representations cannot be directly interpreted by a computer.

The most commonly used software for recognizing chemical-structure images like Kekule or OSRA are based on an optical chemical (structure) recognition. Kekule is a software package developed in the early 1990s, which was designed to read scanned chemical-structure images and interpret them into a formal string representation. It is the chemical equivalent of an OCR (Optical Character Recognition) program. The system involves seven steps: Scanning, Vectorization, Searching for dashed lines and dashed wedges, Character recognition, Graph compilation, Postprocessing and Display and editing. In Kekule, the area of a page that contains a chemical structure diagram needs to be manually identified. Kekule has a built-in algorithm to fix character recognition errors. For this purpose, a neural network is used for generating potential characters with scoring information estimating the likelihood that a specific character corresponds to a certain atom. Even when an incorrectly recognized character has a higher score than the correct candidate, Kekule can fix character-to-atom conversion errors by considering the valence and chemical neighbors of the atom. Still, manual correction at the postprocessing step is often required. A further development to Kekule was OSRA in 2009.

Another cheminformatics tool that is used is CLiDE (Chemical Literature Data Extraction). CLiDE not only aims at extracting chemical structures but also abstracting chemical information from text. By employing the Documental Format Description Language (DFDL) which can describe logical relationships of objects and elements in a document, CLiDE builds logical associations between chemical structures and the text segments of document. Unlike OCR and like Kekule, CLiDE does not have an automated process to discriminate chemical structure diagram from graphical objects, so manual separation of chemical diagrams is necessary. As well as Kekule and OCR, CLiDE requires at least a 300 dpi resolution in scanned images at the scanning step and manual correction at the post processing step to achieve reliable output. However, the drawn chemical structure diagrams are typically embedded in Word documents as GIF or JPG formats, whose the resolution is usually 72-96 dpi. Therefore, these software systems might be impractical tools for fully automated extraction of chemical structure information.

Hence, there is a need for effective image searching capability which could converting the digital raster images of chemical diagrams into representations such as SMILES strings or atom connectivity tables in standard chemical file formats. Once a reliable structure recognition and conversion system is developed it can be used to scan pages of chemical literature and construct a database. The resulting database is one that can be queried with a cheminformatic search engine into which an investigator can input a chemical structure and pull any related information of interest. Novel drug candidates or newly synthesized molecules are usually referenced by chemical structure diagrams rather than molecule names. In addition, a single molecule may have a number of synonyms such that it could be referenced by different names in different articles. Thus, the capability of exploring research articles or patents where the chemical structure or similar compounds are drawn would complement existing text-based search engines for chemical information.

The methods of prior-art documents are based on handwritten rules for extracting chemical structure diagrams in scientific articles, patent documents etc. and convert them to structured representations.

However, a handwritten rule-based classification of structural features in molecular images is unreliable and error-prone. Typical drawing styles of molecular structure follow no standard. Usually just a narrowly defined representation style is possible.

In the recent years, several software programs were developed that could extract chemical structure diagrams in scientific articles and convert them to structured representations.

One such program is described by prior art non-patent literature Molecule Structure Extraction from Document Using Deep Learning by Staker et. al (2019). The method disclosed therein predicts the chemical structure by first encoding the images of the chemical structure into a fixed-length latent space and then decoding using a recurrent neural network.

The present disclosure overcomes the above identified limitations found in the prior art.

The present disclosure provides methods which reliably extracts structured machine-readable chemical structures from templates with diverse formats. Further, the present disclosure relates to methods and apparatuses for extracting a chemical structure using a neural network and machine learning approaches.

Provided are methods and apparatuses for extracting a chemical structure using a neural network. Also, provided are computer-readable media including a program, which when executed by a computer, performs the methods. The present disclosure may address the technical problems addressed above and/or other technical problems not addressed above. As an example, some implementations herein permit a neural network device to extract chemical structures that satisfy various requirements (e.g. identical or equivalent representations). Further, and continuing the example, the neural network device may employ a set of models (e.g., convolutional neural network (CNN) or a detection CNN or a region-based CNN and/or the like) that permits the neural network device to extract chemical structures that satisfy various input requirements. In this way, some implementations herein permit the neural network device to address the technical shortcomings of the related systems, and/or permit the neural network device to extract chemical structures in a more accurate manner, in a more efficient manner, and/or in a manner that conserves computing resources as compared to other systems and techniques that are incapable of doing so.

Additional aspects will be set forth in part in the description which follows and, in part, will be apparent from the description, or may be learned by practice of the presented exemplary embodiments.

According to an aspect of an exemplary embodiment of the present disclosure provided is a method of extracting at least one chemical structure from a template, wherein the method being performed by a neural network device and comprising the steps of receiving an image representation of the template; selecting at least one region of interest from the said image representation, wherein the region of interest comprises at least one chemical structure; segmenting, using a first segmenting algorithm, pixels in the region of interest; creating a first image comprising the pixels related to the chemical structure in the region of interest;

determining, using a neural network detection algorithm, a classification of the first image; constructing from the pixels related to the chemical structure, based on the classification of the first image, part-based image representations of parts of the first image; assembling from the part-based representations an identical representation or an equivalent representation of the chemical structure and, outputting the identical or the equivalent representation of the chemical structure as a structured machine-searchable representation of the chemical structure.

According to an aspect of another exemplary embodiment of the present disclosure provided is a neural network device configured to output a structured machine-searchable representation of chemical structures, the neural network device comprising: an interface configured to receive an image representation of a template comprising at least one chemical structure ; a memory configured to store at least one program; and a processor configure to execute the at least one program to: select at least one region of interest from the said image representation, wherein the region of interest comprises at least one chemical structure; segment, using a first segmenting algorithm, pixels in the region of interest; create a first image comprising the pixels related to the chemical structure in the region of interest; determine, using a neural network detection algorithm, a classification of the first image; construct from the pixels related to the chemical structure, based on the classification of the first image, part-based image representations of parts of the first image; and, assemble, from the part-based representations a graph based representation or a sequence based representation of the chemical structure; output the identical or the equivalent representation of the chemical structure.

According to an aspect of another exemplary embodiment of the present disclosure provided is a method of extracting at least one chemical structure from a template, wherein the method being performed by a neural network device and comprising the steps of: receiving a first image of the template comprising a digital embodiment of the said at least one chemical structure; constructing from the said digital embodiment of the first image, based on the classification of the first image using a neural network detection algorithm, part-based image representations of parts of the first image; assembling from the part-based representations an identical representation or an equivalent representation of the chemical structure; and,; outputting the identical or the equivalent representation of the chemical structure as a structured machine-searchable representation of the chemical structure. According to an aspect of another exemplary embodiment of the present disclosure provided is a method of extracting at least one chemical structure from a template, wherein the method being performed by a neural network device and comprising the steps of receiving a first image of the template comprising a digital embodiment of the said at least one chemical structure constructing from the digital embodiment of the first image, based on at least one classification of the first image using a neural network detection algorithm, part-based image representations of parts of the first image; and, assembling from the part-based representations a graph-based structured machine-searchable representation of the chemical structure..

According to an aspect of another exemplary embodiment of the present disclosure provided is a neural network device configured to output a structured machine-searchable representation of the chemical structure , the neural network device comprising: an interface configured to receive a first image of a template comprising a digital embodiment of at least one chemical structure; a memory configured to store at least one program; and a processor configure to execute the at least one program to receive a first image of the template comprising a digital embodiment of the said at least one chemical structure; construct from the digital embodiment of the first image, based on at least one classification of the first image using a neural network detection algorithm, part-based image representations of parts of the first image; and, assemble from the part-based representations, a graph-based representation of the chemical structure.

According to an aspect of another exemplary embodiment of the neural network device and the method of extracting chemical structure from the a template may further comprise determining the classification of the first image by inputting the first image to the neural network detection algorithm trained on a training data set.

According to an aspect of another exemplary embodiment of the neural network device and the method of extracting chemical structure from the a template may further comprise determining the classification of the first image by inputting the first image to the neural network detection algorithm adapted to detect parts of the first image semantically related to the chemical structure.

According to an aspect of another exemplary embodiment of the neural network device and the method of extracting chemical structure from the a template may further comprise constructing from the pixels related to the chemical structure, based on the classification of the first image using a neural network algorithm, part-based image representations of parts of the first image comprises assigning parts of the first image to at least one member of hierarchical or non-hierarchical categories.

According to an aspect of another exemplary embodiment of the neural network device and the method of extracting chemical structure from the a template may further comprise constructing from the pixels related to the chemical structure, based on the classification of the first image using a neural network algorithm, part-based image representations of parts of the first image comprises assigning categories to the parts of the first image. According to an aspect of another exemplary embodiment of the neural network device and the method of extracting chemical structure from the a template may further comprise constructing from the pixels related to the chemical structure, based on the classification of the first image using a neural network algorithm, part-based image representations of parts of the first image comprises assigning parts of the first image to predefined or categories learnt from a training data set.

According to an aspect of another exemplary embodiment of the neural network device and the method of extracting chemical structure from the a template may further comprise constructing from the pixels related to the chemical structure, based on the classification of the first image using a neural network algorithm, part-based image representations of parts of the first image comprises categorizing the chemically relevant information of the parts of the first image.

According to an aspect of another exemplary embodiment of the neural network device and the method of extracting chemical structure from the a template may further comprise determining, using a neural network detection algorithm, a set of detection bounding boxes from the first image; constructing from the pixels related to the chemical structure, based on classifying each bounding box from the set of detection bounding boxes from the first image using a neural network algorithm, part-based image representations of parts of the first image.

According to an aspect of another exemplary embodiment of the neural network device and the method of extracting chemical structure from the a template may further comprise determining, the classification of the first image using the neural network detection algorithm, which is a convolutional neural network (CNN) or a detection CNN or a region-based CNN (R-CNN) or a Fast R-CNN or a Faster R-CNN or a Mask R-CNN or YoloV3.

According to an aspect of another exemplary embodiment, a non-transitory computer-readable recording medium includes one or more instructions, which when executed by one or more processors, causes the one or more processors to perform operations associated with an exemplary embodiment.

The method of extracting at least one chemical structure from a template according to the present disclosure and the neural network device according to the present disclosure may be used in different real-life applications for example automatically extracting chemical structures from patent literature or non-patent literature. The methods of the present disclosure may also be used in automated processes like creating full or part responses for the office actions to the patent offices or other regulatory authorities.

Furthermore, the methods of the present disclosure may also be used in creating full or in part patent applications.

Although specific advantages have been enumerated above, various embodiments may include some, none, or all of the enumerated advantages.

Other technical advantages may become readily apparent to one of ordinary skill in the art after review of the following figures and description.

It should be understood at the outset that, although exemplary embodiments are illustrated in the figures and described below, the principles of the present disclosure may be implemented using any number of techniques, whether currently known or not. The present disclosure should in no way be limited to the exemplary implementations and techniques illustrated in the drawings and described below.

Unless otherwise specifically noted, articles depicted in the drawings are not necessarily drawn to scale.

Modifications, additions, or omissions may be made to the systems, apparatuses, and methods described herein without departing from the scope of the disclosure. For example, the components of the systems and apparatuses may be integrated or separated. Moreover, the operations of the systems and apparatuses disclosed herein may be performed by more, fewer, or other components and the methods described may include more, fewer, or other steps. Additionally, steps may be performed in any suitable order. As used in this document, "each" refers to each member of a set or each member of a subset of a set.

## BRIEF DESCRIPTION OF THE DRAWINGS

These and/or other aspects will become apparent and more readily appreciated from the following description of the exemplary embodiments, taken in conjunction with the accompanying drawings in which:

    FIG. 1 is a block diagram illustrating a hardware configuration of neural network device according to an exemplary embodiment;
    FIG. 2 is a diagram for describing a computation performed by a neural network according to an exemplary embodiment;
    FIG. 3 is a diagram describing computational steps performed by a region-based convolutional neural network (R-CNN) according to an exemplary embodiment;
    FIG. 4 is a diagram describing computational steps performed by a fast region-based convolutional neural network (Fast R-CNN) according to an exemplary embodiment;
    FIG. 5 is a diagram describing computational steps performed by a faster region-based convolutional neural network (Faster R-CNN) according to an exemplary embodiment;
    FIG. 6 is a diagram describing computational steps performed by a mask region-based convolutional neural network (Mask R-CNN) according to an exemplary embodiment.

## DETAILED DESCRIPTION

Reference will now be made in detail to exemplary embodiments, examples of which are illustrated in the accompanying drawings, wherein like reference numerals refer to like elements throughout. In this regard, the present exemplary embodiments may have different forms and should not be construed as being limited to the descriptions set forth herein. Accordingly, the exemplary embodiments are merely described below, by referring to the figures, to explain aspects. As used herein, the term "and/or" includes any and all combinations of one or more of the associated listed items. Expressions such as "at least one of," when preceding a list of elements, modify the entire list of elements and do not modify the individual elements of the list.

The terms "according to some exemplary embodiments" or "according to an exemplary embodiment" used throughout the specification do not necessarily indicate the same exemplary embodiment.

Some exemplary embodiments of the present disclosure may be represented by functional block configurations and various processing operations. Some or all of these functional blocks may be implemented using various numbers of hardware and/or software components that perform particular functions. For example, the functional blocks of the present disclosure may be implemented using one or more microprocessors or circuits for a given function. Also, for example, the functional blocks of the present disclosure may be implemented in various programming or scripting languages. The functional blocks may be implemented with algorithms running on one or more processors. The present disclosure may also employ conventional techniques for electronic configuration, signal processing, and/or data processing. The terms "mechanism", "element", "unit" and "configuration" may be used in a broad sense and are not limited to mechanical and physical configurations, and may be implemented in hardware, firmware, software, and/or a combination thereof.

Also, connection lines or connection members between the components illustrated in the drawings are merely illustrative of functional connections and/or physical or circuit connections. In actual devices, connections between the components may be represented by various functional connections, physical connections, or circuit connections that may be replaced or added.

Meanwhile, with respect to the terms used herein, template may refer to any executable or non-executable file format with different file extensions. Template may also refer to any image representation of a physical or a virtual document like webpages or scanned images or any other virtual entity from which it is possible to obtain digitalized information regarding the chemical structure(s). The image representation of the template may comprise a complete of a partial section(s) of the physical or the virtual document. The template may also comprise of standard exchange file formats like but not limited to MDL Molfile (*.mol), SDfile (*.sdf), RDfile (*.rdf), SMILES (*.smi), PDB file (*.pdb), CIF (*.cif), JCAMP (*.jdx, *.dx, *.cs), CMS (*.cml) Marvin Documents (*.mrv), Tripos Mol 2 (*.mol2), ChemDraw (*.cdx, *.cdxml), ISIS (*.skc) or Portable Document Format (*.pdf) formats. In addition, an identical or an equivalent representation of the chemical structures may refer to structures like but not limited to structure diagrams, empirical or condensed formula, IUPAC nomenclature for representing the chemical structure or line notations representing the chemical structure as a linear sequence of letters and numbers like Wiswesser Line Notation (WLN), ROSDAL (Representation of Organic Structures Description Arranged Linearly), SMILES (Simplified Molecular Input Line Entry System), Sybyl Line Notation (SLN), or graph-based or matrix-based or a connection table (CT) or a chemical table file, or a SMILES Arbitrary Target Specification (SMARTS) code, an International Chemical Identifier (In-Chi) code, or the like. A condensed formula of a molecule is the formula where the symbols of atoms are listed in order as they appear in the molecule's structure with bond dashes omitted or limited. While vertical bonds are always omitted, sometimes horizontal bonds are included to indicate polyatomic groups. Parentheses in a condensed formula indicate the polyatomic group is attached to the central atom to the left of the parentheses. A true condensed formula can be written on a single line without any branching above or below it.

In addition, classification, may refer to a process of assigning regions or any non-zero number of pixels to at least one member of hierarchical or non-hierarchical categories. Here, the hierarchy of the category is to be understood as a genus and species relationship. For example, positively charged nitrogen as sub class of nitrogen is a hierarchical categorization while considering the hierarchical category of the atoms. The hierarchical or non-hierarchical categories may be pre-defined or may be learnt during the training step. Categories may refer, but are not limited, to chemically irrelevant background, element symbols, isotopes, charges, valences and radicals, abbreviations and aliases like functional groups or alphanumeric identifiers for fragments as used for biochemical polymer description, repeating units, connections or bonds of any kind between atoms and groups, delocalization and aromaticity information, tautomeric states, stereo chemistry labels (like spatial isomers, enantiomers, diastereomers, absolute and relative information, atropisomerism, conformers, rotamers) or any kind of wildcard symbol (Markush structures).

In addition, an artificial neural network (ANN) refers to a collection of fully or partially connected units comprising information to convert input data into output data. In more detail, a convolutional neural network (CNN) refers to a collection of units performing at least once a convolution instead of a general matrix multiplication.

In addition, segmenting refers to a process combining any non-zero number of pixels or regions of an image into at least one segment, optionally applying a pre-defined or machine-learned metrics.

In addition, the term pixels related to a chemical structure, describes the pixel-wise classification result of the input image into chemically meaningful image information and background.

Also, structured data refers to data with any kind of information which is added as meta data to the original data in order to group parts of the original data, facilitating the automatic downstream processing of the resulting information.

Hereinafter, exemplary embodiments of the present disclosure will be described in detail with reference to the accompanying drawings.

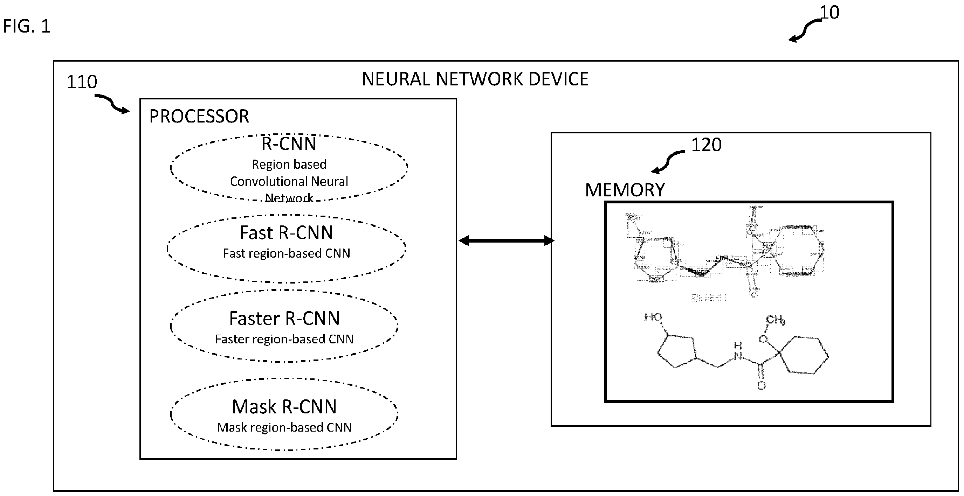

FIG. 1 is a block diagram illustrating a hardware configuration of a neural network device according to an exemplary embodiment.

A neural network device 100 may be implemented using various types of devices such as a personal computer (PC), a server device, a virtual machine, a mobile device, and an embedded device. Examples of the neural network device 100 may include, but are not limited to, a smartphone, a tablet device, an augmented reality (AR) device, an Internet of Things (IoT) device, an autonomous vehicle, a robot, a medical device, and the like which perform speech recognition, image recognition, image classification, and the like using a neural network. Furthermore, the neural network device 100 may be a dedicated hardware (HW) accelerator mounted on the devices described above. The neural network device 100 may be a hardware accelerator such as a neural processing unit (NPU), a tensor processing unit (TPU), and a neural engine, which are dedicated modules for driving a neural network, without being limited thereto.

Referring to FIG. 1, the neural network device 100 includes a processor 110 and a memory 120. FIG. 1 illustrates components of the neural network device 100 related to the exemplary embodiments of the present disclosure. Thus, it should be apparent to a person skilled in the art that the neural network device 100 may further include any other components in addition to the components shown in FIG. 1.

The processor 110 controls the overall function for driving the neural network device 100. For example, the processor 110 controls the overall operation of the neural network device 100 by executing programs stored in the memory **120** of the neural network device **100**. The processor **110** may be implemented as a central processing unit (CPU), a graphics processing unit (GPU), an application processor (APU), virtual CPU (vCPU) and the like provided in the neural network device **100,** without being limited thereto.

The memory **120** is a component, at least partially implemented in hardware, that stores a variety of data processed by the neural network device **100**. For example, the memory **120** may store data to be processed by the neural network device **100**. The memory **120** may also store applications, drivers, and the like to be driven by the neural network device **100.** The memory **120** may include random access memory (RAM) such as dynamic random access memory (DRAM) and static random access memory (SRAM), read-only memory (ROM), electrically erasable programmable read-only memory (EEPROM), CD-ROM, Blue-ray, optical disk storage, hard disk drive (HDD), solid state drive (SSD), flash memory, or the like.

Meanwhile, the memory 120 stores a classification information and a pixel information that are associated with each other as one set. The neural network device 100 may read the classification information and the pixel information from the memory 120 or write the classification information and the pixel information in the memory 120. The pixel information may refer to a normalized version of a set of color- or brightness-encoded values of pixels associated with the chemical structure and may be an integer, a real number or a n-dimensional vector of the before mentioned. In addition, normalization of at least one dimension or on parts of a dimension of the n-dimensional image representation may be performed, and, may comprise any kind of (affine and non-affine) transformation and color/brightness modifications. In addition, the classification information may refer to a one-hot encoded n-dimensional vector. Advantageously, in such a representation of n-dimensional binary vector each position may represent one class and preferably only one class may be set to one. It may also refer to a real-valued vector assigning probability estimates to all potential classes, or a single number indicating the assigned class to a pre-defined and ordered list of all potential classes or the like.

The processor 110 may drive an artificial neural network (ANN), Convolutional Neural Network (CNN or ConvNet), region-based CNN (R-CNN), Fast R-CNN, Faster R-CNN, or You Only Look Once (YOLO) type of object detection algorithms.

The processor 110 may allow the ANN to learn by using an image of the chemical structure and may determine a set of bounding box detections, each associated with a part of the chemical structure. The bounding box detection set comprises the localization information of the bounding box and a corresponding class label. By driving the trained ANN, the processor 110 may perform a computation using the image of the chemical structure or its digital embodiment as input data to an input layer and generate a set of bounding box detections, each associated with a part of the chemical structure and each comprised of the localization of the bounding box and a corresponding class label as output data based on a computation result.

The processor 110 may allow the CNN to learn by using the image of the chemical structure as derivable from a template and may determine a set of bounding box detections, each associated with a part of the chemical structure. The bounding box detection set comprises the localization information of the bounding box and a corresponding class label. By driving the trained CNN, the processor 110 may perform a computation using a template comprising a chemical structure as input data to an input layer and may provide output class label and bounding box data based on a computation result.

Meanwhile, the neural network device 100 may further include a user interface (not shown). The user interface refers to a device used to input data to control the neural network device 100. Examples of the user interface may include, but are not limited to, a key pad, a dome switch, a touch pad (e.g., capacitive overlay type, resistive overlay type, infrared beam type, surface acoustic wave type, integral strain gauge type, and piezo electric type), a jog wheel, and a jog switch.

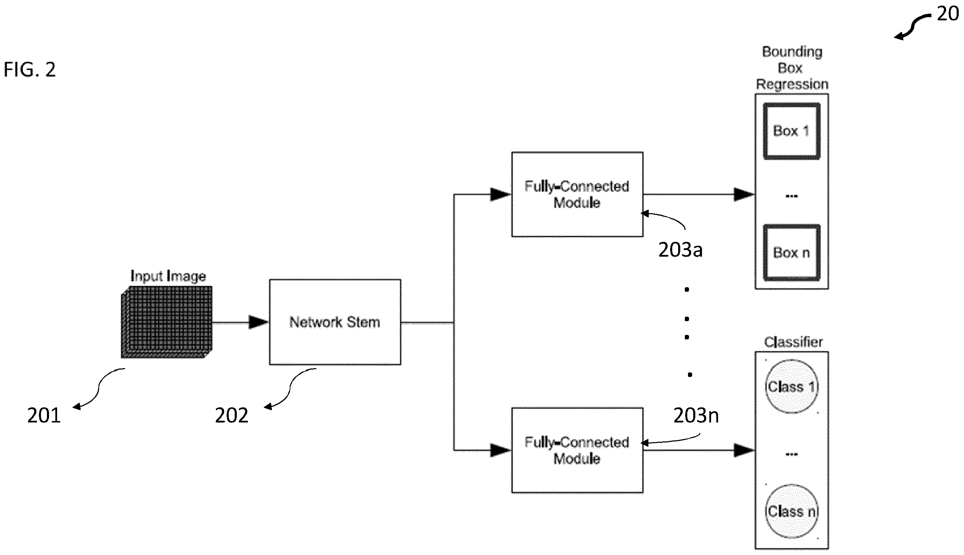

Hereinafter, methods of extracting a chemical structure by using the neural network device 100 and evaluating the extracted chemical structure will be described in detail. The methods to be described below may be performed by the processor 110 and the memory 120 of the neural network device 100. FIG. 2 is a diagram for describing a computation performed by a convolutional neural network CNN according to an exemplary embodiment with hidden layers.

In contrast to other deep learning structures, CNN 20 may extract features of small portions of input images, which are called receptive fields. At each convolutional layer of this model, the input data is convolved with a set of filters, each generating a new feature map of the input data. Next, these features are subject to a non-linear transformation. In some embodiments according to the present disclosure non-linear transformation may be combined with a normalization layer. The same process is repeated for the remaining convolutional layers. In order to increase the receptive field of subsequent convolutional layers and to make it invariant to small local deformations in the input, pooling layers may also be incorporated in CNN 20 in order to aggregate feature values of pixel neighborhoods by using typically the max or mean operation. Fully connected layers may also be added at the end of the convolutional pipeline, feeding the activations in the final layer through a softmax function. CNN 20 may comprise of several convolutional filters and many convolutional and pooling layers to enable the network to integrate the information extracted by different filters and various levels of abstraction. Since the CNN 20 may include many layers to process valid information, that is to transform their input by applying convolution filters, the CNN 20 may process data in a more complex way as compared to a neural network including a single layer. The parameters of CNN 20 that could be trained may be determined by using gradient-based optimization or the like. The hyperparameters of CNN 20, for example but not limited to architecture design parameters, that is the number of layers or the number filters per layer or the like, may be determined using Bayesian optimization or the like.

Referring to FIG. 2, the CNN 20 performs the extraction of the chemical structure from the templates by performing classification and detection. CNN 20 may include an input (not shown) to receive an input image 201. The CNN 20 performs the task of detecting bounding boxes on the input image representation of the template and categorizing each bounding box into different classes labels. The network stem 202 extracts high-level features from the inputted image. This may be performed by running several convolutions and pooling operations on the inputted image. The convolutional layers perform convolutions to detect features. The convolutional layers serve as feature extractors, and thus they learn the feature representations of their inputted image. The neurons in the convolutional layers may be arranged into feature maps. Each neuron in a feature map has a receptive field, which is connected to a neighborhood of neurons in the previous layer via a set of trainable weights, sometimes referred to as a filter. Inputs to a convolutional layer are convolved with the learned weights in order to compute a new feature map, and the convolved results are sent through a nonlinear activation function and optionally a normalization function. All neurons within a feature map have weights that are constrained to be equal; however, different feature maps within the same convolutional layer have different weights so that several features can be detected at each location. The $k$th output feature map $Yk$ can be computed as

$$Yk = f\left(Wk * x\right)$$

where the input image is denoted by $x$; the convolutional filter related to the kth feature map is denoted by $Wk$; the multiplication sign in this context refers to the 2D convolutional operator, which is used to calculate the inner product of the filter model at each location of the input image; and $f(\cdot)$ represents the nonlinear activation function, optionally together with a normalization function. Nonlinear activation functions for example but not limited to sigmoid or hyperbolic tangent functions or rectified linear units allow for the detection of nonlinear features. Normalization functions for example but not limited to batch normalization or group normalization functions allow to robustify and speed up the training process. The network stem 202 may also comprise of pooling layers. Pooling layers reduce the spatial resolution of the feature maps and achieve spatial invariance to input distortions and translations. In some embodiments, average pooling aggregation layers propagate the average of all input values within a small neighborhood of a feature map to the next layer. In some embodiments, max pooling aggregation layers propagate the maximum value within a receptive field to the next layer. Several convolutional and pooling layers may be stacked on top of each other to detect features representations in moving through the network. The fully connected layers or modules that follow these layers interpret these feature representations and perform the function of high-level reasoning.

The network stem 202 may be followed by some fully-connected modules 203a...203n which connects the network stem 202 to the output layer. The fully-connected modules may combine classification of a region of the inputted image with localization. For localization the object is located within the image. This location is typically specified by a two-dimensional bounding box.

Further, the CNN **20** performs a computation based on received input data (e.g., hand $I_2$), and generates output data (e.g., $O_1$ and $O_2$) based on a computation result.

For example, , the CNN **20** may include an input layer (Layer 1), two hidden layers (Layer 2 and Layer 3), and an output layer (Layer 4). Since the CNN **20** may include many layers to process valid information, the CNN **20** may process more complex data as compared to a neural network including a single layer. Meanwhile, the CNN **20** is merely an example and may also include more or less layers and more or less channels. That is, the CNN **20** may have various structures of layers different from described above.

Each of the layers included in the CNN 20 may have a plurality of channels. The channels may correspond to a plurality of artificial nodes known as neurons, processing elements (PEs), units, or similar terms. For example, Layer 1 may include two channels (nodes), and Layers 2 and 3 may include three channels, respectively. However, the layers are merely examples and each of the layers included in the CNN 20 may have various numbers of channels (nodes).

The channels included in each of the layers of the CNN 20 may be interconnected as a directed acyclic graph to process data. For example, a channel may perform a computation of data received from channels of one layer and output a computation result to channels of another layer.

Input and output of each channel may be referred to as input activation and output activation. That is, an activation may be not only an output of one channel but also a parameter corresponding to an input of channels included in a successive layer. Meanwhile, each of the channels may determine an activation thereof based on activations and weights received from channels included in a previous layer. The weight is a parameter used to calculate the output activation of each channel.

The CNN 20 may perform a computation using template comprising at least one chemical structure as input data to the input layer and generate a classification that is class labels associated with a part of the chemical structure. Further, the CNN 20 may optionally also generate a set of bounding box detections, each associated with a part of the chemical structure, that may comprise the localization of the bounding box and a corresponding class label as output data based on a computation result. Alternative embodiments of CNN 20 or variants thereof might also output a segmentation masks for each detection. Thus, a part-based representation of the chemical structure may be constructed comprising a distribution of categories (or class labels) and bounding boxes and possibly mask segmentations is obtained.

The chemical structure may be extracted from the part-based representation based on the classified structural entities in the image representation of the chemical structure by assembling a graph-based structure for example, but not limited to, by adding at least one atom by class label and bounding box. Optionally, chemical structure may be extracted from the part-based presentation based on the classified structural entities in the image representation of the chemical structure by assembling mask position, checking mask. In some embodiments, the extraction of the chemical structure may be achieved by bounding box overlap and/or proximity and/or orientation with bonds for each pair of atoms or any number preselected pairs of atoms may be creating bonds according to class label, orientation and direction of the bonds or the like.

In addition, the recovered structure can be globally or locally assigned with a confidence value, indicating the likelihood of the correctness of the recognized structure diagram or parts of it. Optionally, the extracted chemical structure can be sanitized and/or converted into structured and unstructured output formats.

Hereinafter, descriptions given above with reference to FIG. 2 will not be repeated for descriptive convenience.

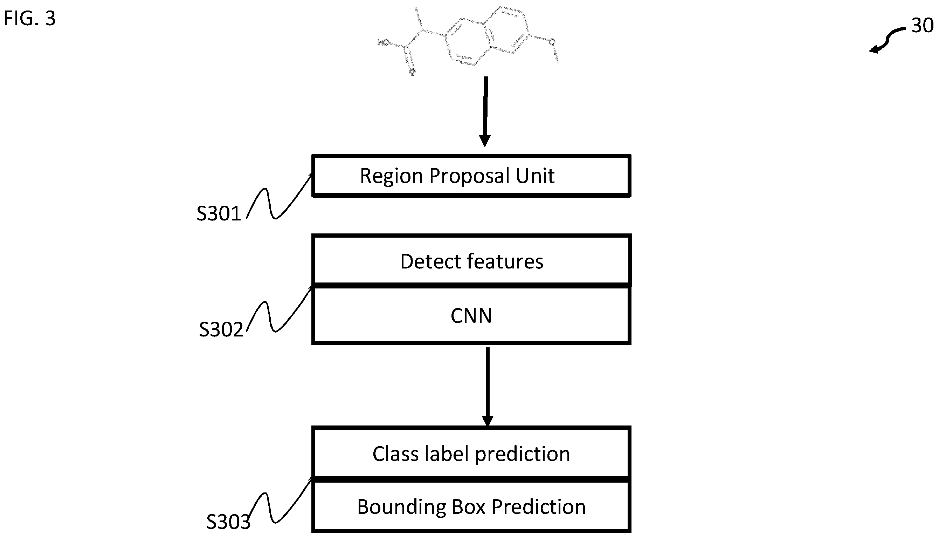

FIG. 3 is a diagram for describing a computation steps performed by a region-based CNN (R-CNN) neural network device 30 according to an exemplary embodiment. FIG. 3 describes the steps of a neural network device according to the present disclosure using a region-based CNN 30. However, the present disclosure is not limited to a particular region-based CNN as described herein.

A R-CNN 30 is a neural network that fulfills the purpose of object detection that is detection of the parts of a chemical structure. That is, the R-CNN 30 is a neural network in which objects are localized with a CNN, which may be trained to detect the objects with only a small quantity of annotated detection data. R-CNN 30 localizes the parts of chemical structures from the templates by operating within the "recognition using regions" paradigm, for object detection. R-CNN 30 is trained by supervised pre-training on a large auxiliary dataset, followed by fine-tuning with a domain-specific dataset. In alternative embodiments the R-CNN may be trained on the domain-specific dataset.

R-CNN 30 may include an input layer for receiving a template (not shown). R-CNN 30 may use a segmenting algorithm or a region proposal algorithm for example, but not limited to, Hierarchical Grouping algorithm, Selective Search algorithm or the like to identify some number of bounding-box object region candidates ("region of interest" or "RoI"). R-CNN 30 may use a CNN or the like to identify some features from each region that allow for classification and bounding box regression. In other words, R-CNN 30 detect objects or the parts of the chemical structure without the intermediate region proposal step

R-CNN 30 may be illustrated as comprising of two or three modules. However, such a representation is merely intended for the purposes of explaining the region-based neural network device according to an exemplary embodiment. It should not be read as distinctly separate essential features of the region-based CNN neural network device 300 according to the exemplary embodiment. Nor is R-CNN 30 limited to a stepwise approach or a limitation that the modules function as independent entities.

A region proposal unit 301 may propose category-independent regions in the inputted image representation of the chemical structure. The region proposal unit 301 may use region proposal method like objectness, selective search, category-independent object proposals, constrained parametric min-cuts (CPMC), multi-scale combinatorial grouping or the like. The region-based CNN neural network device 30 is agnostic to the particular region proposal method. These proposed regions are generally selected on multiple scales and have different shapes and sizes. The category and ground-truth bounding box of each proposed region is labeled. A feature selection unit 302 may use a pre-trained CNN for detecting features from the proposed regions. The pre-trained CNN is selected and placed, in truncated form, before the output layer. It may transform each proposed region and may use forward computation to output the features detected from the proposed regions. Further, the features and labeled category of each proposed region are combined for training the classifying unit 303. The classifying unit 303 determines a class label and a bounding box. For that, it may comprise of a class label prediction module and a bounding box regression module or a module that may combine both functionalities, As an example, multiple support vector machines or the like may be used for object class label prediction. Here, each support vector machine may be used to determine whether an example belongs to a certain category. For bounding box prediction, as an example, a linear regression model may be used to determine the coordinates of the ground-truth bounding box.

Referring to FIG. 3, a region-based CNN (R-CNN) neural network device 30 performs the following steps as shown therein for extracting the chemical structure. In step S301, the region proposal unit 301 performs a selective search on the template comprising an image representation of the chemical structure. The region proposal unit 301 proposes category independent regions. FIG. 3 only shows some category independent regions. In some alternative embodiments, the region proposal unit 301 of the region-based CNN (R-CNN) neural network device 30 may propose any number category independent regions. In step S302, the feature selection unit 302 may use a pre-trained CNN for detecting features (segmenting) from the proposed regions. The feature selection unit 302 in step S302 may recognize features based on visual cues within the proposed regions that are semantically related to the chemical structure. The classifying unit 303 in step S303 may classify the proposed regions of the chemical structure based on the recognized features. Class labels stem from hierarchical or non-hierarchical categories for example, but not limited to, structural entities related to molecular topology like adjacent regions of bonds and atoms in the chemical structure, or parts of the chemical structure having positive or negative charges and valence information or wedged/dashed/unspecified/aromatic/cis/trans/coordinated bonds of any order of the chemical structures having the same topology (constitution) but different topography (geometry) also known as stereoisomers, or condensed sum formula or polymer like depictions of the chemical structure including parentheses and alphanumeric symbols or structure-like diagrams. The classification can be carried out for example, but not limited to, with chemically relevant information of the chemical structure like knowledge of the atomic coordinates and/or the orientation of the bonds with respect to the atoms in the chemical structure or the like.. The region proposal bounding boxes are refined by, for example, a linear regression model that is trained using the detected CNN features and ground truth bounding boxes.

Thus, a part-based representation of the chemical structure is constructed comprising a distribution of categories (or class labels), bounding boxes is obtained.

The chemical structure may be extracted from the part-based presentation based on the classified structural entities in the image representation of the chemical structure by assembling a graph-based structure for example, but not limited to, by adding at least one atom by class label and bounding box

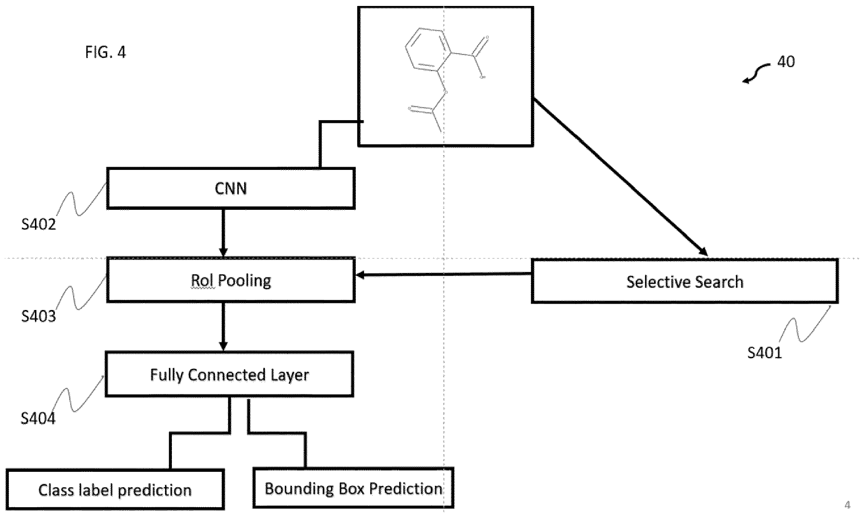

While segmenting thousands of proposed regions from a single digital image of the template for the advantageous benefits of the computation speed, a Fast region based CNN (Fast R-CNN) neural network device 40 R-CNN may be used to extract the chemical structures for a digital embodiment of a template comprising a large number of chemical structures as shown in FIG. 4.

FIG. 4 is a diagram for describing the computation steps performed by Fast R-CNN neural network device 40 according to an exemplary embodiment.

The Fast R-CNN neural network device 40 performs CNN-based feature extraction on the template comprising chemical structures as a whole different from the region-based feature extraction of the R-CNN neural network device 30.

Hereinafter, descriptions given above with reference to Figures. 2 and 3 will not be repeated for descriptive convenience.

Referring to FIG. 4, in step S401, selective search proposes n proposed regions with different shapes indicative of regions of interests (RoIs) in the template comprising chemical structures on the output of CNN. Also, in Step S402, the entire template comprising chemical structures is inputted to CNN input for feature detection, rather than each proposed region. Fast R-CNN neural network device 40 introduces RoI pooling, which in step S403 uses the CNN output from the step S402 and proposed RoIs from the template comprising chemical structure as input from S401 to output a spatially rescaled representation of the features detected from each proposed region. RoI pooling layer in Step S403 in Fast R-CNN is different from a normal pooling layer of a CNN. In normal pooling layer pooling window, padding, and stride are set to control the output shape. In an RoI pooling layer of step S403, the output shape of each region may be specified, for example by specifying the height and width of each region, and input features are resampled and pooled to fit the specified output shape. Further, fully connected layers are used in step S404 to transform the pooled RoI features . The output of the fully connected layer, is inputted to a final softmax layer (not shown) which predicts the class label (categories) while another linear regression layer predicts a bounding box refinement like S303. In this manner, the category and bounding box for each proposed region is predicted.

The chemical structure is extracted in a manner similar to as described in the embodiment related to region-based neural network of FIG. 3 and is not repeated here merely for the purposes of descriptive convenience.

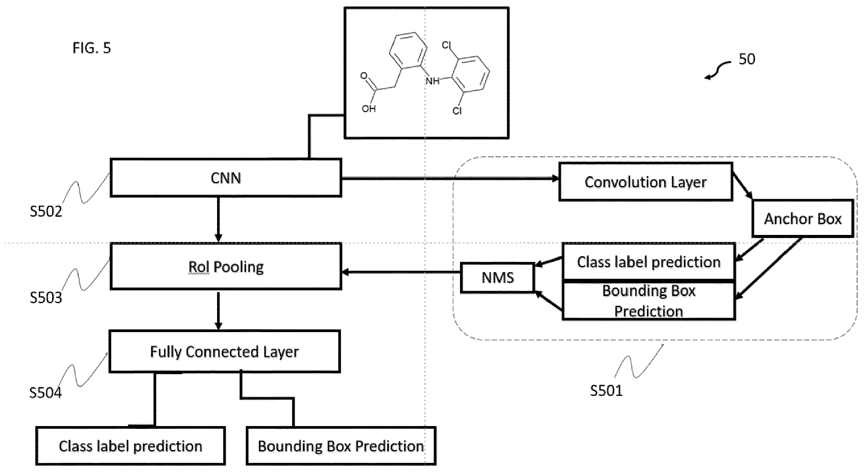

For further benefits of the computational speed, Faster R-CNN is described in FIG.5 of the present disclosure. FIG. 5 is a diagram for describing a computation performed by Faster R-CNN neural network device 50 according to an exemplary embodiment. Faster CNN advantageously reduces the number of proposed regions generated by using a region proposal network and hence enhances the computational speed.

FIG. 5 is a diagram for describing the computation steps performed by Faster R-CNN neural network device 50 according to an exemplary embodiment.

Hereinafter, descriptions given above with reference to Figures. 2 to 4 will not be repeated for descriptive convenience. In particular, Faster R-CNN only changes the method for segmenting proposed regions from selective search of the previous embodiments to a region proposal network. The other parts of neural network device 40 remain unchanged and hence will not be repeated for descriptive convenience. The extraction of the chemical structure from the classified image representation also remains the same.

Referring to FIG. 5 in step S501 including multiple sub steps as shown by the dashed box a region proposal network uses convolutional layers to transform the CNN output. In this manner, the region proposal network may use an intermediate image feature map that is different from the output of the CNN. Further, a fixed set of anchor boxes is applied to each element in the intermediate feature map to create multiple putative bounding boxes of different sizes and aspect ratios. The features of the elements at the center on the bounding boxes are used to predict the binary category (object or background) and to refine bounding box coordinates relative to their respective anchor boxes. A non-maximum suppression (NMS) is used to remove similar bounding box results that correspond to category predictions of the structural entities of the chemical structure. The predicted bounding boxes are outputted as the proposed regions required by the RoI pooling layer for step S503.

In step S501, the region proposal network as a part of the Faster R-CNN model, is also trained together with the rest of the neural network. Thus, the region proposal network can learn how to generate high-quality proposed regions, which reduces the number of proposed regions while maintaining the precision of object detection.

If training data is labeled with the pixel-level positions of each object in template, advantageously a Mask R-CNN model can effectively use these detailed labels to further improve the precision of object detection.

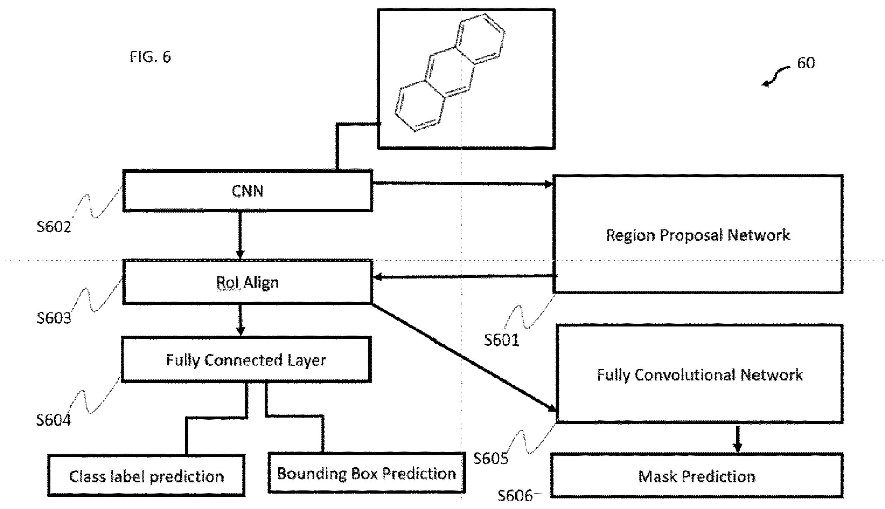

FIG. 6 is a diagram for describing a computation performed by Mask R-CNN neural network device 60 according to an exemplary embodiment.

Hereinafter, descriptions given above with reference to Figures. 2 to 5 will not be repeated for descriptive convenience. In particular, Mask R-CNN only changes the method for the step related to region of interests pooling layer (RoI pooling layer) of the previous embodiments 4 and 5 to RoI alignment layer and adds a mask prediction module. RoI alignment layer advantageously uses bilinear interpolation to retain spatial information on feature maps, making Mask R-CNN better for pixel-level predictions in the template comprising the chemical structure. The other parts of neural network device 50 remain unchanged and hence will not be repeated for descriptive convenience. In addition to the positional information of bounding boxes and their content's classification, masks may be used during the construction of the part-based molecular representation for determining by pixel-wise overlap the connections of molecular elements for example, but not limited to, bonds, atoms and before mentioned classes. Referring to FIG. 6, in step S603, RoI alignment layer Mask R-CNN neural network device 60 outputs feature maps of the same shape for all RoIs. This has the advantage of not only predicting the categories for classification and bounding boxes of RoIs,but allowing to use an additional fully convolutional network (FCN) to predict the pixel-level positions of objects in the template comprising the chemical structure. A fully convolutional network (FCN) uses convolutional layers to transform image pixels to pixel categories. The FCN transforms the height and width of the intermediate layer feature map back to the size of input image through the transposed convolution layer, so that the predictions have a one-to-one correspondence with input image in spatial dimension (height and width). Given a position on the spatial dimension, the output of the channel dimension will be a category prediction of the pixel corresponding to the location.

Fully-convolutional network can also be used with any of the above embodiments as described in the present disclosure and is not specifically limited to the Mask R-CNN model as disclosed in embodiment 6.

In some embodiments, the above objective may be achieved by applying one or more machine learning based neural network methods that process their input data according to a learned (supervised) set of rules and map the input data to a set of parts, where each part comprises of a bounding box, a distribution over class labels with corresponding confidence level, and a pixel segmentation mask. In some embodiments, the above objectives may be achieved by applying one or more machine learning methods that process their input data according to a self-learned (unsupervised) set of rules.

Also, the aforementioned exemplary embodiments may be embodied in the form of a recording medium including instructions executable by a computer, such as a program module, executed by a computer. The computer-readable medium may be any recording medium that may be accessed by a computer and may include volatile and non-volatile media and removable and non-removable media. The computer-readable medium may include a non-transitory computer-readable medium that stores one or more instructions that, when executed by one or more processors, cause the one or more processors to perform operations associated with exemplary embodiments described herein. Also, the computer-readable medium may include computer storage media and communication media. The computer storage media include volatile and non-volatile and removable and non-removable media implemented using any method or technology to store information such as computer-readable instructions, data structures, program modules, or other data. The communication media include computer-readable instructions, data structures, program modules, or other data in a modulated data signal, or other transport mechanisms and include any delivery media.

In addition, throughout the specification, the term "unit" may be a hardware component such as a processor or a circuit and/or a software component executed by the hardware component such as a processor.

The above description of the present disclosure is provided for the purpose of illustration, and it should be understood by those skilled in the art that various changes and modifications may be made without changing technical conception and essential features of the present disclosure. Thus, it is clear that the above-described illustrative exemplary embodiments are illustrative in all aspects and do not limit the present disclosure.

For example, each component described to be of a single type may be implemented in a distributed manner. Likewise, components described to be distributed may be implemented in a combined manner.

It should be understood that exemplary embodiments described herein should be considered in a descriptive sense only and not for purposes of limitation. Descriptions of features or aspects within each exemplary embodiment should typically be considered as available for other similar features or aspects in other exemplary embodiments.

While one or more exemplary embodiments have been described with reference to the figures, it will be understood by those of ordinary skill in the art that various changes in form and details may be made therein without departing from the spirit and scope as defined by the following claims.

## Claims

1. A method of extracting at least one chemical structure from a template, wherein the method being performed by a neural network device and comprising the steps of:

   receiving an image representation of the template;
   selecting at least one region of interest from the said image representation, wherein the region of interest comprises at least one chemical structure;
   segmenting, using a first segmenting algorithm, pixels in the region of interest;
   creating a first image comprising the pixels related to the chemical structure in the region of interest;
   determining, using a neural network detection algorithm, a classification of the first image;
   constructing from the pixels related to the chemical structure, based on the classification of the first image using a neural network algorithm, part-based image representations of parts of the first image; and,
   assembling from the part-based representations an identical representation or an equivalent representation of the chemical structure; and,
   outputting the identical or the equivalent representation of the chemical structure as a structured machine-searchable representation of the chemical structure.

2. A method of extracting at least one chemical structure from a template, wherein the method being performed by a neural network device and comprising the steps of:

   receiving a first image of the template comprising a digital embodiment of the said at least one chemical structure;
   constructing from the said digital embodiment of the first image, based on the classification of the first image using a neural network detection algorithm, part-based image representations of parts of the first image; and,
   assembling from the part-based representations an identical representation or an equivalent representation of the chemical structure; and,
   outputting the identical or the equivalent representation of the chemical structure as a structured machine-searchable representation of the chemical structure.

3. The method as claimed in any one of the preceding claim 1 or 2, further comprising:
   constructing, from the first image, based on the classification of the first image using a neural network detection algorithm, part-based image representations of parts of the first image comprises creating parts of a graph-based representation or parts of a sequence-based representation of the chemical structure.

4. A method of extracting at least one chemical structure from a template, wherein the method being performed by a neural network device and comprising the steps of:

   receiving a first image of the template comprising a digital embodiment of the said at least one chemical structure;
   constructing from the digital embodiment of the first image, based on at least one classification of the first image using a neural network detection algorithm, part-based image representations of parts of the first image; and,
   assembling from the part-based representations a graph-based structured machine-searchable representation of the chemical structure.

5. The method as claimed in any one of the preceding claims 1 to 4 further comprising:
   determining the classification of the first image by inputting the first image to the neural network detection algorithm trained on a training data set.

6. The method as claimed in any one of the preceding claims 1 to 4, further comprising:
   determining the classification of the first image by inputting the first image to the neural network detection algorithm adapted to detect parts of the first image semantically related to the chemical structure.

7. The method as claimed in any one of the preceding claims 1 to 4, further comprising:
constructing from the pixels related to the chemical structure, based on the classification of the first image using a neural network algorithm, part-based image representations of parts of the first image comprises assigning categories to the parts of the first image.

8. The method as claimed in any one of the preceding claims 1 to 4, further comprising:
constructing from the pixels related to the chemical structure, based on the classification of the first image using a neural network algorithm, part-based image representations of parts of the first image comprises assigning parts of the first image to predefined or categories learnt from a training data set.

9. The method as claimed in any one of the preceding claims 1 to 4, further comprising:
constructing from the pixels related to the chemical structure, based on the classification of the first image using a neural network algorithm, part-based image representations of parts of the first image comprises categorizing the chemically relevant information of the parts of the first image.

10. The method as claimed in any of the preceding claims, further comprising:
determining, the classification of the first image using the neural network detection algorithm, which is a convolutional neural network (CNN) or a detection CNN or a region-based CNN (R-CNN) or a Fast R-CNN or a Faster R-CNN or a Mask R-CNN.

11. The method as claimed in claim 1, further comprising:
segmenting, using the first segmenting algorithm, which is an artificial neural network (ANN), pixels in the region of interest.

12. A neural network device configured to output a structured machine-searchable representation of chemical structures , the neural network device comprising:

an interface configured to receive an image representation of a template comprising at least one chemical structure ;
a memory configured to store at least one program; and a processor configure to execute the at least one program to:

select at least one region of interest from the said image representation, wherein the region of interest comprises at least one chemical structure;
segment, using a first segmenting algorithm, pixels in the region of interest; create a first image comprising the pixels related to the chemical structure in the region of interest;
determine, using a neural network detection algorithm, a classification of the first image; construct, from the pixels related to the chemical structure, based on the classification of the first image using a neural network algorithm, part-based image representations of parts of the first image; and, assemble, from the part-based representations an identical representation or an equivalent representation of the chemical structure; and,

output, the identical or the equivalent representation of the chemical structure as a structured machine-searchable representation of the chemical structure.

13. The neural network device according to claim 12 wherein the processor is further configured to execute the said program to carry out the steps of any one of the method of claims 5 to 11.

14. A neural network device configured to output a structured machine-searchable representation of the chemical structure , the neural network device comprising:

an interface configured to receive a first image of a template comprising a digital embodiment of at least one chemical structure;
a memory configured to store at least one program; and,
a processor configure to execute the at least one program to carry out the steps of any of the method of claims 4 to 10.

15. A computer-readable medium comprising instructions which, when executed by a computer, cause the computer to carry out the steps of any one of the method of claims 1 to 11.

16. A computer program comprising instructions which, when the program is executed by a computer, cause the computer to carry out the steps of any one of the method of claims 1 to 11.

FIG. 1

NEURAL NETWORK DEVICE

110 — PROCESSOR
- R-CNN — Region based Convolutional Neural Network
- Fast R-CNN — Fast region-based CNN
- Faster R-CNN — Faster region-based CNN
- Mask R-CNN — Mask region-based CNN

120 — MEMORY

HO, CH₃

10

EP 3 876 236 A1

FIG. 2

FIG. 3

30

Region Proposal Unit

S301

Detect features

CNN

S302

Class label prediction

Bounding Box Prediction

S303

EP 3 876 236 A1

16

FIG. 4

Selective Search — S401

CNN — S402

RoI Pooling — S403

Fully Connected Layer — S404

Bounding Box Prediction

Class label prediction

FIG. 5

FIG. 6

Europäisches
Patentamt

European
Patent Office

Office européen
des brevets

# EUROPEAN SEARCH REPORT

**Application Number**

EP 20 16 1025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | PARK J. ET AL: "Automated extraction of chemical structure information from digital raster images", CHEMISTRY CENTRAL JOURNAL, BIOMED CENTRAL LTD, LO, vol. 3, no. 1, 5 February 2009 (2009-02-05), page 4, XP021052480, ISSN: 1752-153X, DOI: 10.1186/1752-153X-3-4 * the whole document * | 1-16 | INV. G16C20/70 G16C20/80 |
| X | GKOUTOS G. V. ET AL: "Chemical Machine Vision: Automated Extraction of Chemical Metadata from Raster Images", JOURNAL OF CHEMICAL INFORMATION AND COMPUTER SCIENCES, vol. 43, no. 5, 1 September 2003 (2003-09-01), pages 1342-1355, XP055735521, US ISSN: 0095-2338, DOI: 10.1021/ci034017n * the whole document * | 1-16 | |
| X | KRALLINGER M. ET AL: "Information Retrieval and Text Mining Technologies for Chemistry", CHEMICAL REVIEWS, vol. 117, no. 12, 5 May 2017 (2017-05-05), pages 7673-7761, XP055392300, US ISSN: 0009-2665, DOI: 10.1021/acs.chemrev.6b00851 * paragraph [4.3.] * * table 6 * | 1-16 | |

TECHNICAL FIELDS SEARCHED (IPC)

G16C

-/--

The present search report has been drawn up for all claims

| Place of search | Date of completion of the search | Examiner |
|---|---|---|
| The Hague | 2 October 2020 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 20 16 1025

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FLUCK J. ET AL: "Information extraction technologies for the life science industry", DRUG DISCOVERY TODAY: TECHNOLOGIES, ELSEVIER, AMSTERDAM, NL, vol. 2, no. 3, 1 October 2005 (2005-10-01), pages 217-224, XP005118460, ISSN: 1740-6749, DOI: 10.1016/J.DDTEC.2005.08.013 * page 221, left-hand column, line 12 - right-hand column, line 28 * | 1-16 | |
| A | BUKHARI S. S. ET AL: "Chemical Structure Recognition (CSR) System: Automatic Analysis of 2D Chemical Structures in Document Images", 2019 INTERNATIONAL CONFERENCE ON DOCUMENT ANALYSIS AND RECOGNITION (ICDAR), IEEE, 20 September 2019 (2019-09-20), pages 1262-1267, XP033701489, DOI: 10.1109/ICDAR.2019.00-41 [retrieved on 2020-01-31] * the whole document * | 1-16 | |
| A | THARATIPYAKUL A. ET AL: "ChemEx: information extraction system for chemical data curation", BMC BIOINFORMATICS, vol. 13, no. S17, 1 December 2012 (2012-12-01), page S9, XP055735539, DOI: 10.1186/1471-2105-13-S17-S9 * the whole document * | 1-16 | TECHNICAL FIELDS SEARCHED (IPC) |

The present search report has been drawn up for all claims

| Place of search | Date of completion of the search | Examiner |
|---|---|---|
| The Hague | 2 October 2020 | Godzina, Przemyslaw |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2